(19) Europäisches Patentamt

European Patent Office

Office européen des brevets

(11) Veröffentlichungsnummer: **0 494 615 A1**

## (12) EUROPÄISCHE PATENTANMELDUNG

(21) Anmeldenummer: **92100093.1**

(51) Int. Cl.5: **A61K 49/00**

(22) Anmeldetag: **04.01.92**

(30) Priorität: **09.01.91 DE 4100470**

(43) Veröffentlichungstag der Anmeldung:
**15.07.92 Patentblatt 92/29**

(84) Benannte Vertragsstaaten:
**PT**

(71) Anmelder: **BYK GULDEN LOMBERG CHEMISCHE FABRIK GMBH**
**Postfach 100310, Byk-Guldenstrasse 2**
**W-7750 Konstanz(DE)**

(72) Erfinder: **Beller, Klaus-Dieter**
**Franz-Moser-Strasse 5**
**W-7750 Konstanz 16(DE)**
Erfinder: **Linder, Rudolf**
**Felchengang 22**
**W-7750 Konstanz 16(DE)**

(54) **Echokontrastmittel.**

(57) Eine wäßrige Zubereitung zur Aufnahme und Stabilisierung von Mikrogasbläschen zur Verwendung als Echokontrastmittel enthaltend Polyoxyethylenpolyoxypropylen-Polymere und negativ geladene Phospholipide wird angegeben, die sich zur Linksherzdarstellung eignet.

EP 0 494 615 A1

## Technisches Gebiet

Die Erfindung betrifft eine wäßrige Zubereitung zur Aufnahme und Stabilisierung von Mikrogasbläschen zur Verwendung als Echokontrastmittel.

## Stand der Technik

Da Ultraschall von in Flüssigkeiten suspendierten Gasbläschen stark reflektiert wird, wurden als Kontrastmittel für die Ultraschalldiagnostik schon frühzeitig wäßrige Zubereitungen vorgeschlagen, die stabilisierte Mikrogasbläschen enthalten. Mikrogasbläschen lassen sich in wäßrigen Zubereitungen durch Herabsetzung der Oberflächenspannung, d.h. durch Zusatz geeigneter Tenside stabilisieren.

In der EP-B-0077752 wird angegeben, daß wäßrige Lösungen, die ein Tensid oder ein Tensidgemisch und zusätzlich eine viskositätserhöhende Substanz enthalten, vorteilhafte kontrasterzeugende Eigenschaften aufweisen. Als in Frage kommende Tenside werden u.a. nicht ionogene Lecithine und Lecithinfraktionen sowie Polyoxyethylenpolyoxypropylen-Polymere angegeben. In den sechs Herstellungsbeispielen der EP-B-0077752 werden Zubereitungen angegeben, die als Tensid jeweils ein Polyoxyethylenpolyoxypropylen-Polymeres und als viskositätserhöhende Substanz Glucose oder Dextran oder das Polyoxyethylenpolyoxypropylen-Polymere selbst enthalten. Eine Nacharbeitung der Herstellungsbeispiele hat gezeigt, daß die kontrastgebende Wirkung nicht befriedigend ist. So eignen sich die Zubereitungen der EP-B-0077752 nicht für Linksherzdarstellungen.

Es wurde nun überraschenderweise gefunden, daß wäßrige Zubereitungen, die neben Polyoxyethylenpolyoxypropylen-Polymeren negativ geladene Phospholipide enthalten, sich in überragender Weise zur Aufnahme und Stabilisierung von Mikrogasbläschen eignen.

## Beschreibung der Erfindung

Gegenstand der Erfindung sind daher wäßrige Zubereitungen zur Aufnahme und Stabilisierung von Mikrogasbläschen zur Verwendung als Echokontrastmittel enthaltend Polyoxyethylenpolyoxypropylen-Polymere und negativ geladene Phospholipide.

Weitere Gegenstände ergeben sich aus den Patentansprüchen.

Als Polyoxyethylenpolyoxypropylen-Polymere werden solche mit einem mittleren Molekulargewicht von 8350 bis 14000 bevorzugt. Polyoxyethylenpolyoxypropylen-Polymere werden auch als Poloxamere bezeichnet und sind z.B. unter dem Warenzeichen Pluronics® (Wyandotte Chemicals Corp.) im Handel erhältlich. Die erfindungsgemäßen Zubereitungen enthalten 0,1 bis 10 %, vorzugsweise 1 bis 5 %, Polyoxyethylenpolyoxypropylen-Polymere. Die negativ geladenen Phospholipide sind in einer Menge von 0,01 bis 5 %, vorzugsweise 0,5 bis 2 % enthalten. Prozentangaben beziehen sich jeweils auf Gewicht/Volumen.

Als negativ geladene Phospholipide kommen Phosphatidylglycerole, Phosphatidylinositole, Phosphatidylethanolamine und Phosphatidylserine und deren Lysoformen in Frage. Unter Lysoformen der negativ geladenen Phospholipide werden negativ geladene Phospholipide verstanden, die nur einen Acylrest enthalten. Bevorzugt sind Lysoformen der negativ geladenen Phospholipide, bei denen die Acylgruppe am Sauerstoff des Kohlenstoffatoms 1 des Glycerinmoleküls gebunden ist. Besonders bevorzugte negativ geladene Phospholipide sind Dipalmitoylphosphatidylglycerol (DPPG) und Distearoylphosphatidylglycerol (DSPG), wobei Distearoylphosphatidylglycerol (DSPG) ganz besonders bevorzugt ist.

Die erfindungsgemäßen Zubereitungen zeichnen sich gegenüber dem Stand der Technik dadurch aus, daß mit geringem mechanischen Aufwand Mikrogasbläschen enthaltende Echokontrastmittel erzeugt werden können, die wegen ihrer großen Stabilität einen lang anhaltenden Kontrast erzeugen und sich ausgezeichnet auch zur Linksherzdarstellung eignen. Besonders hervorzuheben ist, daß sich die erfindungsgemäßen Zubereitungen ausgezeichnet zur Darstellung von inneren Oberflächenstrukturen eignen, da die Mikrogasbläschen anscheinend gut an Oberflächen haften und so auch nach Ausspülen der im Lumen von Gefäßen befindlichen Mikrogasbläschen einen aufschlußreichen Kontrast erzeugen. Dadurch ist es beispielsweise möglich, die Dynamik des Herzens auch nach Auswaschen des Kontrastmittels deutlicher darzustellen.

Die Herstellung der erfindungsgemäßen Zubereitungen ist unproblematisch und kann erfolgen, indem die einzelnen Komponenten zusammen oder nacheinander in Wasser eingetragen und nötigenfalls unter Erwärmen und Rühren gelöst werden. Gewünschtenfalls kann noch sterilisiert werden, beispielsweise durch Hitzesterilisation.

Als besonders gut geeignet zur Einstellung der Isotonie der erfindungsgemäßen Zubereitungen haben sich Glycerin, Mannit und Ammoniumsalze von Aminosäuren, vorzugsweise Glycin, erwiesen.

Die Erzeugung der Mikrogasbläschen erfolgt zweckmäßigerweise erst kurz vor Verabreichung an die zu untersuchenden Patienten und wird auf an sich bekannte Weise vorgenommen. Wird z.B. die erfindungsgemäße Zubereitung in einer Durchstechflasche zur Verfügung gestellt, so kann die Lösung zusammen mit der gewünschten Menge Luft in eine übliche Spritze aufgezogen werden und wieder mit möglichst hohem Druck über eine enge Kanüle in die Durchstechflasche eingespritzt werden. Nötigenfalls wird das Aufziehen und Ausspritzen mehrmals wiederholt. Alternativ können die erfindungsgemäßen Zubereitungen auch zwischen zwei Spritzen über ein Verbindungsstück mit engem Querschnitt oder eine zwischen die beiden Spritzen geschaltete Mischkammer hin- und hergedrückt werden. Das letztgenannte Verfahren führt zu besonders informativen Ultraschallbildern, wobei gleichzeitig die Ergiebigkeit weiter erhöht wird.

Als Gase zur Erzeugung der Mikrogasbläschen kommen alle physiologisch verträglichen Gase in Frage. Die erfindungsgemäßen Zubereitungen werden pro 1 ml mit 0,01 bis 0,1, vorzugsweise mit 0,04 bis 0,06 ml Gas aufgeschäumt. Sie werden nach Erzeugung der Mikrogasbläschen vorzugsweise intravenös verabreicht. Je nach Anwendungszweck werden 1 bis 20 ml, vorzugsweise 2 bis 8 ml, und besonders bevorzugt 5 ml der erfindungsgemäßen Zubereitungen verabreicht.

Besonders hervorzuheben ist, daß die erfindungsgemäßen Zubereitungen wegen ihrer im Vergleich zum Stand der Technik erhöhten Ergiebigkeit niedriger dosiert werden können.

Beispiele

1. 3,0 g Polyoxyethylenpolyoxypropylen-Polymeres mit einem mittleren Molekulargewicht von 8400 (Pluronic®F68), 1,0 g Dipalmitoylphosphatidylglycerol (DPPG) und 3,6 g Glycerin werden in 80 ml Wasser eingetragen. Man erwärmt auf ca. 80°C und rührt, bis eine vollständige Auflösung stattgefunden hat. Nach dem Abkühlen wird mit destilliertem Wasser auf 100 ml aufgefüllt.

2. Man verfährt wie in Beispiel 1, mit dem Unterschied, daß statt DPPG 1,0 g Sojaphosphatidylglycerol (Fa. Lucas Meyer, Hamburg) verwendet wird.

3. In 80 ml Wasser werden 1,1 g Glycin eingetragen. Mit verdünntem Ammoniak und wird ein pH von 6 bis 7 eingestellt. In die Lösung werden 3,0 g Polyoxyethylenpolyoxypropylen-Polymeres mit einem mittleren Molekulargewicht von 8400 (Pluronic®F68) und 1,0 g DPPG gegeben. Man erwärmt auf ca. 80°C und rührt bis eine vollständige Auflösung stattgefunden hat. Nach dem Abkühlen wird mit destilliertem Wasser auf 100 ml aufgefüllt.

4. Man verfährt wie in Beispiel 3, mit dem Unterschied, daß statt DPPG 1,0 g Sojaphosphatidylglycerol (Fa. Lucas Meyer) verwendet wird.

5. In 80 ml Wasser werden 4,0 g Polyoxyethylenpolyoxypropylen-Polymeres (Poloxamer 188, Pluronic® F68), 1,0 g Distearoylphosphatidylglycerol und 5,4 g Mannit eingetragen. Man erwärmt auf ca. 80°C und rührt bis zur vollständigen Auflösung. Nach dem Abkühlen wird mit destilliertem Wasser auf 100 ml aufgefüllt.

Vergleichsversuche

Die Untersuchungen wurden an wachen männlichen Beagle-Hunden (18,2-30,5 kg Körpergewicht) durchgeführt. Den Hunden wurden jeweils 5 ml i.v. der nachfolgend beschriebenen Kontrastmittelzubereitungen appliziert:

A: Eine pro 1000 ml 35 g vernetzte Polypeptide enthaltende Infusionslösung zur Plasmasubstitution (Haemaccel® der Fa. Behringwerke)

B: Echovist® (Echokontrastmittel der Fa. Schering)

C: Eine wässrige Lösung enthaltend 4 Gew.-% Poloxamer 188 (Pluronic® F68) und 4 Gew.-% Glucose (Beispiel 1 in EP 0 077 752)

D: Eine wässrige Lösung enthaltend 2 Gew.-% Poloxamer und 4 Gew.-% Glucose (Beispiel 2 in EP 0 077 752)

E: Eine wässrige Lösung enthaltend 1 Gew.-% Poloxamer und 4 Gew.-% Glucose (Beispiel 3 in EP 0 077 752)

F: Erfindungsgemäße Zubereitung nach Beispiel 5

Die Lösungen A, C, D, E und F werden luftfrei in einer ersten Spritze aufgezogen. Diese Spritze wird sodann mit dem freien Ende einer fest mit einer zweiten Spritze verbundenen, 0,18 ml Luft enthaltenden Mischkammer konnektiert. Unmittelbar vor der Applikation werden die Lösungen fünfmal aus der ersten Spritze über die Mischkammer in die zweite Spritze und wieder zurück gepumpt.

Das handelsübliche Kontrastmittel B wird nach den Anweisungen der Packungsbeilage bereitet.

Die echokardiographischen Ultraschallaufnahmen wurden mit einem Ultraschallgerät Sonoscope 4 mit mechanischem Kopf bei 3,5 MHz durchgeführt. Die Videoprints der erhaltenen Ultraschallbilder wurden bezüglich der Kontrastintensität densitometrisch ausgewertet. Das verwendete Densitometer (Gretag D182) bestimmt die Änderungen der Helligkeit (brightness) in 100 Schritten in einem Bereich von 0,00 bis 2,50 Dichteeinheiten (density units). Die Kalibrierung erfolgt anhand der herstellerseitig bereitgestellten Kalibrierungskarte nach DIN 16536 (calibration reference), wobei dem hellsten Weiß der Wert 1,64 und dem dunkelsten Schwarz der Wert 0,00 zugeordnet wird. Das Mittel aus vier einzelnen Bestimmungen auf einer Fläche von 1 cm x 1 cm ergibt den Wert für jedes Tier für die applizierte Zubereitung.

Die erhaltenen Ergebnisse sind in der nachfolgenden Tabelle wiedergegeben.

| | rechter Ventrikel | | | linker Ventrikel | | |
|---|---|---|---|---|---|---|
| 5 ml | Kontrast | Intensität max | 10sec | Kontrast | Intensität max | 10sec |
| A | ja | 1,18 | 0,86 | nein | 0,00 | 0,00 |
| B | ja | 1,09 | 0,65 | nein | 0,00 | 0,00 |
| C | ja | 1,20 | 0,78 | nein | 0,00 | 0,00 |
| D | ja | 1,23 | 0,87 | nein | 0,00 | 0,00 |
| E | ja | 1,22 | 0,93 | nein | 0,00 | 0,00 |
| F | ja | 1,19 | 0,82 | ja | 0,78 | 0,72 |

Intensität in Density Units (DU)

Aus den Ergebnissen ergibt sich, daß die erfindungsgemäßen Echokontrastmittel im Gegensatz zu den Echokontrastmitteln nach dem Stand der Technik lungengängig sind und daher sich ausgezeichnet zur Linksherzdiagnostik eignen. Die Anwendbarkeit der Ultraschallbildgebung in der Herzdiagnostik wird durch die erfindungsgemäßen Echokontrastmittel erheblich erweitert.

Darüberhinaus wurde gefunden, daß die Mikrobläschen der erfindungsgemäßen Echokontrastmittel anscheinend eine erhebliche Affinität zu den inneren Oberflächen von Gefäßen und Hohlräumen des Körpers aufweisen. Das hat zur Folge, daß die Umrisse von Gefäßen und Hohlräumen sehr viel besser und damit informativer dargestellt werden als dies mit Kontrastmitteln nach dem Stand der Technik möglich war. Besonders vorteilhaft ist hierbei, daß diese stark verbesserte Darstellung der Oberflächen von Gefäßen und Hohlräumen auch noch bestehen bleibt, wenn das Lumen des Gefäßes oder Hohlraumes bereits frei von Echokontrastmittel ist. Diese überraschende Kontrastierung von Oberflächen läßt sich zum Beispiel zur Beobachtung des Endocards nutzen.

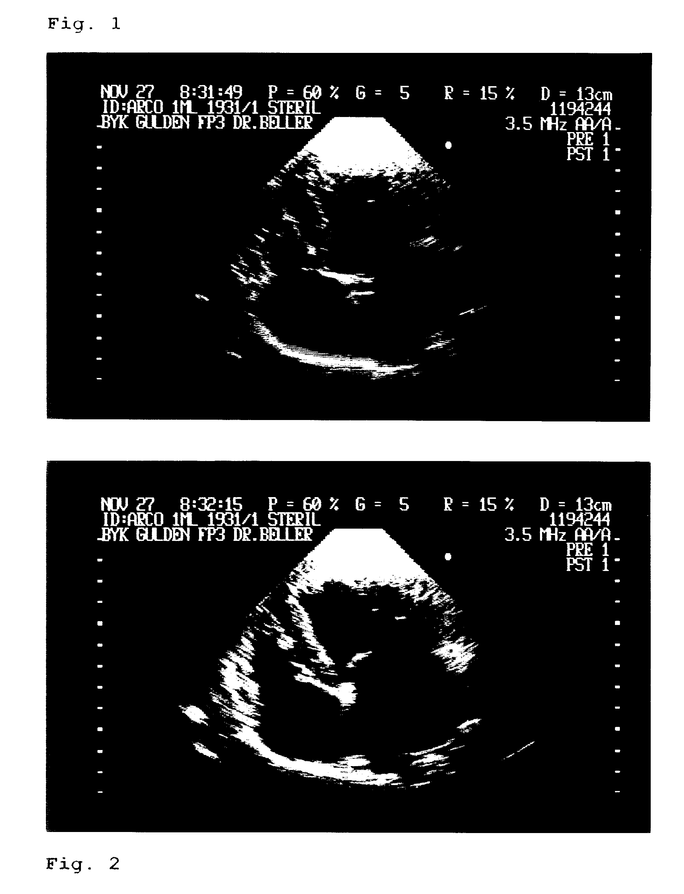

In den Abbildungen 1 und 2 ist das Ergebnis eines Versuches zur Belegung dieser neuartigen Kontrastierung von Oberflächenstrukturen dargestellt.

Abbildung 1 zeigt das echokardiographische Bild des Endocards eines wachen Beagle-Hundes im sogenannten Vierkammerblick unmittelbar vor Erscheinen des ersten Kontrastes nach Verabreichung von 1 ml Echokontrastmittel nach Beispiel 1.

Abbildung 2 zeigt das Endocard des Tieres, nachdem das Echokontrastmittel bereits wieder aus dem Herzen ausgewaschen war.

Aus dem Vergleich der beiden Abbildungen ergibt sich, daß mit den erfindungsgemäßen Echokontrastmitteln eine unerwartete Zeichnung des Endocards möglich ist, die für diagnostische Zwecke einen hohen Informationsgewinn bedeutet.

**Patentansprüche**

**1.** Wäßrige Zubereitung zur Aufnahme und Stabilisierung von Mikrogasbläschen zur Verwendung als Echokontrastmittel enthaltend Polyoxyethylenpolyoxypropylen-Polymere und negativ geladene Phospholipide.

2. Zubereitung nach Anspruch 1, dadurch gekennzeichnet, daß Polyoxyethylenpolyoxypropylen-Polymere mit einem mittleren Molekulargewicht von 8350 bis 14000 verwendet werden.

3. Zubereitung nach Anspruch 1, dadurch gekennzeichnet, daß Polyoxyethylenpolyoxypropylen-Polymere in einer Menge von 0,1 bis 10 % (Gewicht/Volumen) enthalten sind.

4. Zubereitung nach Anspruch 3, dadurch gekennzeichnet, daß die Polyoxyethylenpolyoxypropylen-Polymere in einer Menge von 1 bis 5 % (Gewicht/Volumen) enthalten sind.

5. Zubereitung nach Anspruch 1, dadurch gekennzeichnet, daß als negativ geladene Phospholipide Phosphatidylglycerole, Phosphatidylinositole, Phosphatidylethanolamine und/oder Phosphatidylserine enthalten sind.

6. Zubereitung nach Anspruch 5, dadurch gekennzeichnet, daß als negativ geladenes Phospholipid Distearoylphosphatidylglycerol enthalten ist.

7. Zubereitung nach Anspruch 1, dadurch gekennzeichnet, daß negativ geladenes Phospholipid in einer Menge von 0,01 bis 5 % (Gewicht/Volumen) enthalten ist.

8. Zubereitung nach Anspruch 1, dadurch gekennzeichnet, daß sie 3 % (Gewicht/Volumen) Polyoxyethylenpolyoxypropylen-Polymere mit einem mittleren Molekulargewicht von 8400 und 1 % (Gewicht/Volumen) Distearoylphosphatidylglycerol enthält.

9. Zubereitung nach Anspruch 1, dadurch gekennzeichnet, daß das oder die negativ geladenen Phospholipide als Lysoform zugegen sind.

10. Verfahren zur Herstellung einer wäßrigen Zubereitung zur Aufnahme und Stabilisierung von Mikrobläschen zur Verwendung als Echokontrastmittel, dadurch gekennzeichnet, daß man Polyoxyethylenpolyoxypropylen-Polymere zusammen mit einem negativ geladenen Phospholipid und für zur Erreichung der Isotonie üblichen Hilfsstoffe in Wasser löst.

Fig. 1

Fig. 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | EP-A-0 318 081 (AKZO N.V.) <br> * das ganze Dokument * <br> --- | 1-10 | A61K49/00 |
| D,Y | EP-A-0 077 752 (SCHERING A.G.) <br> * Ansprüche 1-17 * <br> --- | 1-10 | |
| Y | WORLD PATENTS INDEX LATEST <br> Section Ch, Week 8421, <br> Derwent Publications Ltd., London, GB; <br> Class A, AN 84-131234 <br> & JP-A-59 067 229 (GREEN CROSS CORP) 16. April 1984 <br> * Zusammenfassung * <br> --- | 1-10 | |
| P,Y | FILE SERVER STN KARLSRUHE,FILE BIOSIS <br> ABSTRACT NO.92:8782; BA93:8782 <br> & INT.J.PHARM.(AMST.) VOLUME 75 (2-3) <br> PAGES 171-180, 30 SEP.1991 <br> * Zusammenfassung * <br> ----- | 1-10 | |
| | | | **RECHERCHIERTE SACHGEBIETE (Int. Cl.5)** <br><br> A61K |

Der vorliegende Recherchenbericht wurde für alle Patentansprüche erstellt

| Recherchenort | Abschlußdatum der Recherche | Prüfer |
|---|---|---|
| DEN HAAG | 27 APRIL 1992 | SITCH W.D.C. |